# EUROPEAN PATENT APPLICATION

(11) **EP 1 749 550 A1**
(43) Date of publication of application: **07.02.2007**
(21) Application number: 05291664.0
(22) Date of filing: 04.08.2005
(51) Int. Cl.: A61N 5/10

(54) **Method and apparatus for applying radiotherapy**

(71) Applicant: Institut Curie, 75005 Paris (FR)
(72) Inventor: Ferrand, Régis, 78000 Versailles (FR); Meyroneinc, Samuel, 75014 Paris (FR); Mammar, Hamid, 91190 Gif Sur Yvette (FR); Rosenwald, Jean-Claude, 94400 Vitry Sur Seine (FR); Mazal, Alejandro, 94400 Vitry Sur Seine (FR)
(74) Representative: Pontet, Bernard

(57) **Abstract**

The present invention relates to methods and apparatuses for applying radiotherapy.

The method comprises detection of markers and of bad markers to precisely determine localization and or orientation of the patient at the treatment zone (30) in relation to the central axis beam (14). The apparatus comprises a robot (10) which is controlled to localize and/or orientate the patient to be treated in relation with recognition of the actual markers.

Radiotherapy.

## Description

Instant invention relates to methods and apparatuses for positioning a body such as a living body and their application to radiotherapy.

In order to treat some tumors of the human body, it is well-known to expose them to predetermined radiations, such as X-rays and, more recently, proton beams.

A positioning system has been proposed which comprises an industrial robot having six degrees of freedom. A support for a patient, like a chair or a couch, is mechanically coupled to the wrist of the robot arm and a learning process is applied to prepare movement of the patient from a docking position to a treatment position. Such a prior art has been illustrated in a Conference Paper, entitled *"*Integrating an industrial robot and multi-camera computer vision systems into a patient positioning system for high-precision radiotherapy", in the Name of E.A. de Kock et alii, pronounced at ISR-2004 Conferences, Paris, March 24, 2004.

Unfortunately, use of radiation beams as in protontherapy, raises many difficult problems. Proton beams are produced from a particle accelerator, namely a proton-synchrotron, which has many drawbacks. Firstly, it is costly due to the heavy mass and volume of the equipment, the use of rare components and services. Therefore, any protontherapy device should be able to treat a sufficiently high number of patients to reduce induced costs for each individual treatment. Secondly, exposure to the vicinity of the beam is extremely severe. Therefore, it is highly recommended that no service agent be inside a security area around the patient under treatment. Thirdly, it is well-known that high-energy radiation beams are very noxious for any healthy living tissue around the precise location of the targeted tumor or body part. Therefore, it is necessary to ensure both highly precise positioning and orientation of the patient in relation with the radiated beam. Fourthly, in most cases, the patient will be submitted to a succession of treatment sessions and it is important that the patient be replaced back each time in the right position with respect to the beam in a simple, fast and non-costly way.

An object of the invention is to improve the methods and apparatuses for positioning a body, for example in a context of a radiotherapy treatment.

According to the invention, a method is provided for positioning a living body, which comprises:
- measuring a location of a plurality of positioning items; and
- determining from reference locations of the positioning items and the measured locations if at least one of the positioning items moved with respect to at least another one.

The method of the invention could also show any of the following features:
- the locations are measured with respect to a given referential, the body being moveable with respect to this referential;
- determining if at least one of the positioning items moved with respect to the others;
- if one of the positioning items moved, determining which it is and positioning the living body by using the positioning items except this one;
- for at least one of the pairs of the positioning items, comparing the distance between their measured positions and the distance between their reference positions;
- making the comparison for all the positioning pairs of items;
- for at least a predetermined group of positioning items, calculating a value which takes into account for each item the distance between its measured position and its reference position, and determining if this value is above a predetermined threshold;
- for the group or at least one of the groups, the number of items in the group is less than the total number of positioning items;
- calculation and determination are made for a plurality of the groups so that each positioning item belongs to groups comprising different combinations of positioning items;
- some of the values being above their corresponding threshold and some others not, determining if at least one of the items belongs to groups associated with the above value without belonging to the other groups, and if yes, determining which it is;
- after the body has been moved, the measuring and determining steps are performed again with the previously measured locations used as reference locations replacing the former reference locations; and
- both determination steps are performed on the same day or on different days.

According to the invention, we also provide a device for positioning a living body, comprising means for measuring a location of a plurality of positioning items, and means for determining from reference locations of the positioning items and the measured locations if at least one of the positioning items moved with respect to at least another one.

According to the invention, we also provide an apparatus for moving a living body, comprising a device according to the invention.

According to the invention, we also provide a non invasive method for positioning a living body, comprising setting a prosthesis including at least a marker to both X-rays and nuclear magnetic resonance, into at least one natural body cavity.

The method of the invention could also show any of the following features:
- the prosthesis is formed according to the proper shape of the said body cavity, personalized for each patient anatomical features in order to ensure a good reproducibility of positioning between each treatment session;
- the prosthesis or at least one of the prosthesis belongs to the following group: a mouth prosthesis, a nose prosthesis, and an ear prosthesis;
- the prosthesis or at least one of the prosthesis comprises at least two markers spaced from each other; and
- the number of prosthesis set onto the body is at least 2, 3 or 4.

According to the invention, we also provide a prosthesis shaped to be set into a natural cavity of a living body and including at least a marker to both X-rays and nuclear magnetic resonance.

According to the invention, we also provide a method for controlling a device arranged to move a body, such as a living body, between initial and final positions by deforming the device along one among a plurality of possible configurations enabling this movement, in which the deformation configuration is chosen according to at least one cinematic feature of the configuration.

The method of the invention could also show any of the following features:
- the feature or at least one of the features is chosen from the group consisting of:
   - a level of risks;
   - a maximum acceleration;
   - a maximum speed; and
   - a number of degree of freedom involved by the movement.
- the device is controlled so that the body remains in a predetermined area smaller than any area comprising all the possible locations of the body moved by the device.

According to the invention, we also provide an apparatus arranged for moving a body, such as a living body, between initial and final positions by deforming itself along one among a plurality of possible configurations enabling this movement, the apparatus comprising control means which enable to choose the deformation configuration according to at least one cinematic feature of the configuration.

According to the invention, we also provide an apparatus for moving a body with respect to a base of the apparatus, the apparatus comprising holding means for holding the body during a movement and comprising at least two different items, at least one of which being arranged to carry the body, the apparatus being arranged to detachably fix any of the items to the holding means.

Any of the methods of the invention could also show any of the following features:
- at least one two-dimensional image of the body is taken; and
- determining three dimensional coordinates of some points of the body.

According to the invention, we also provide a treatment method, such as a method for treating a tumor by radiotherapy, which comprises a method according to the invention.

Finally any of the apparatus of the invention could also show any of the following features:
- it comprises means for treating a tumor by radiotherapy; and
- it comprises a gantry.

A preferred embodiment of the invention is hereinafter described with reference to the accompanying drawings, in which:
- Figure 1 shows a top view of a treatment room built specially to perform the method according to the preferred embodiment of the invention with an apparatus according to the invention;
- Figures 2 and 3 are lateral views of an industrial robot which is part of the apparatus according to the preferred embodiment of the invention of figure 1, on which are affixed two respective means for carrying the body of a patient to be treated;
- Figure 4 is a perspective view of the robot of figures 2 or 3;
- Figure 5 is a schematic view of both steps for acquiring images before and during treatment;
- Figure 6 shows both lateral and front views of means for forming a treatment beam;

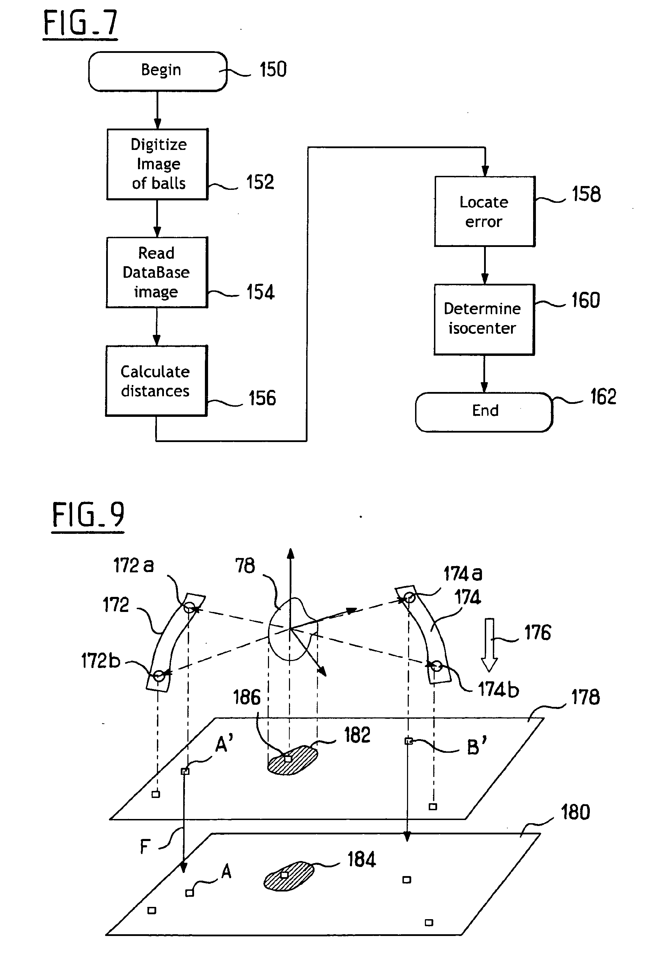

- Figures 7 to 8 are flowcharts showing different steps of the method of the invention according to the preferred embodiment;
- Figure 9 shows a schematic perspective view illustrating a step of the method in which a missing or displaced marker is detected; and
- Figure 10 is a schematic lateral view of an alternative embodiment of the apparatus of the invention.

### The Room

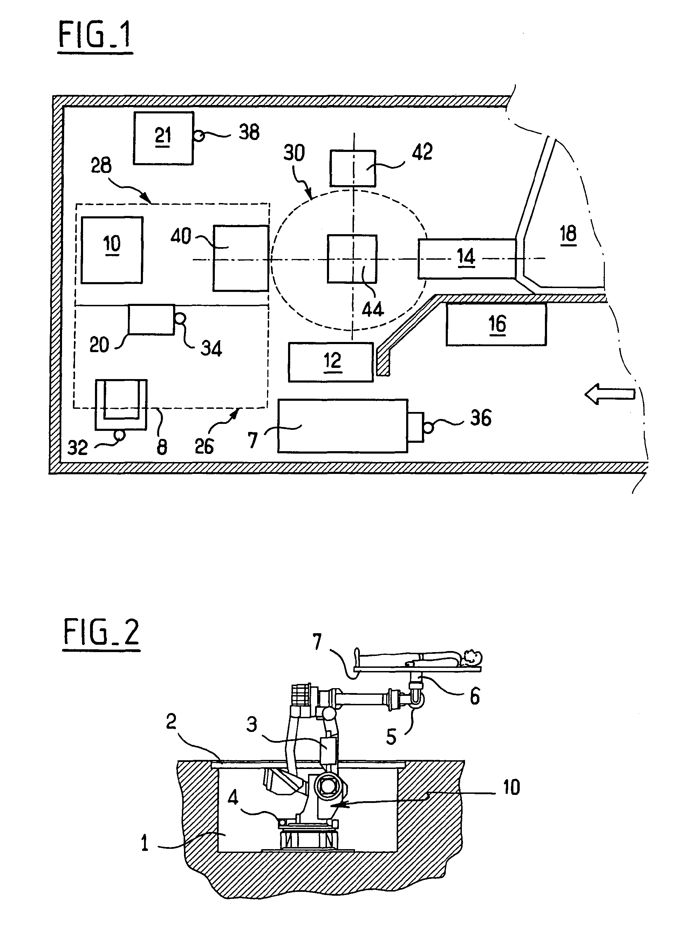

Figure 1 illustrates a room where is performed the method according to the preferred embodiment of the invention. The treatment room is built with convenient materials to ensure radioprotection at the vicinity of a particle generator 18. The generator comprises an elongate tube 14 which enters inside the treatment room. The axis of this tube serves as a reference axis for movements and treatments of patient inside the room.

The room comprises two parts:
- an entrance which has been marked with an arrow indicating the path of an incoming patient.;
- a treatment part in which treatment of a patient is run.

Both parts are separated by an angulated wall shown between elements 12 and 16 which are described below and a door (not shown but located at the place of an element 36 which is also described below).

The room is separated from its environment by walls and the two parts of the treatment room are also well separated from each other by the wall, all of which have been drawn with hatched lines. The entrance is protected against radio emission by the wall when treatment is performed.

The treatment part of the room comprises:
- a first zone in which are located some supports for a patient like a chair 8 and a couch 7;
- a second zone 26 in which a robot installed in a pit 10 is located; and
- a third zone limited with a line 30 in which the contended patient will be treated with at least one particle beam radiated from tube 14.

A docking station 20 which comprises a static part affixed to the ground of the treatment room is provided in the second zone. Docking station 20 carries a reference part 34 and fixture means which match with corresponding reference parts and fixture means of both patient supports 7 and 8. The robot also carries corresponding reference parts and fixture means formed to match with those of the patient support means.

A phantom element formed by a tank 21 is provided with its proper reference part and fixing means 38 formed to match with those of the robot. Phantom tank 21 comprises a container filled with water or any convenient reference material both for control of the treatment beam and x-ray imaging means which will be described hereinafter.

Around the third zone 30 are located means 40, 42, 44 for X-ray imaging along one or two different axis. Imaging means are operative during a setting step of the method as it appears below.

A station 12 is provided at the door between entrance and treatment parts, which includes electronic and electrical means for controlling robot 10 and imaging means 40, 42 and 44. During a setting step of the method, at least one operator controls the robot and imaging means at the station 12 acting on a terminal (not shown) to prepare treatment of the patient. To ensure control of the robot, some programs are run to prepare each sequence of movements.

Before the treatment begins, the operator should go back to the entrance part of the room in front of a terminal of processing means 16 which helps him to control and fires the treatment, patient being positioned by the industrial robot to ensure a secure and precise treatment with the at least one particle beam radiated from tube 14.

### The Robot

At figure 2, robot 10 is showed which comprises an arm 3 presenting six degrees of freedom. Robot is settled in pit 1 which is covered and protected with a lid or cover 2 which is built so that the robot arm 3 rises above lid 2. No opening is visible on the lid. Therefore, nobody could fall in. To ensure such a result, the lid 2 comprises a rotating part which rotates on pit 1 with the movement of the arm 3 of the robot and a translating part which follows movements of arm 3 by sliding in the rotating part of the lid.

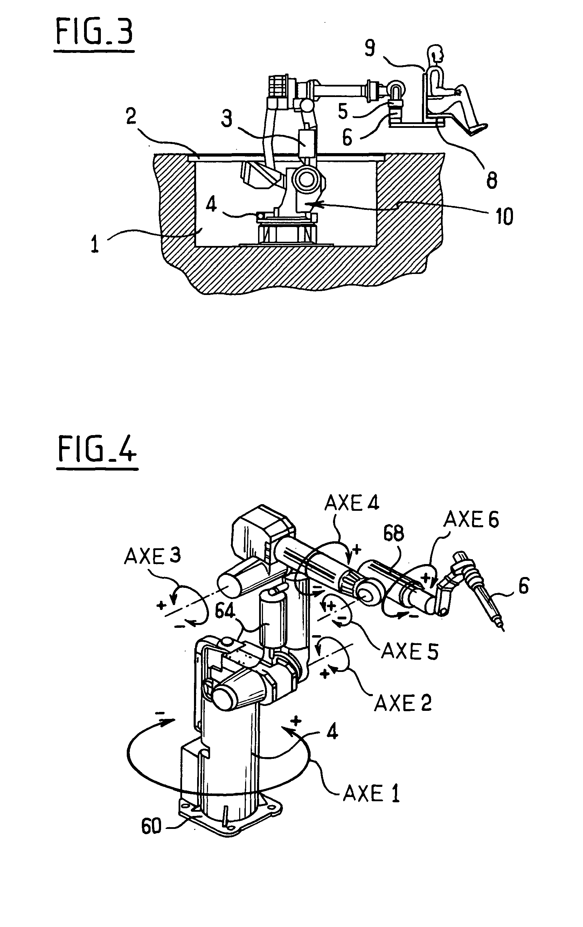

As showed on figure 2, a base 4 of the robot is affixed on the ground. The other linear sections of the arm are connected by joints until a terminal wrist 5 which is equipped with a fixture part 6 which engages with the coupling part of the support means like the couch 7 illustrated on figure 2 or the chair of figure 3. Indeed, at figure 3 is illustrated another support means for patient in the form of a chair 8 with a backseat 9. At figures 2 and 3 same elements are marked with same reference numerals.

To enhance security during movements of arm 3, each part of the arm which could be in contact with objects or persons is provided with contact detectors which are electrically connected with the means implemented in station 12 for controlling the movement of the arm. In instant case, each contact detector is built under an elongated shape comprising a lever mounted on at least two springs and an electrical contactor which changes its electrical state when the lever is pushed when any contact on its length occurs.

The same applies for any lateral part of any of the patient support means, namely the chair 8 or the couch 7. Precisely, a periphery of them is provided with such detectors for signaling to the control means any accidental contact.

Figure 4 more precisely shows the industrial robot with its six axes, AXE 1 to AXE 6, with a terminal fixture part 6 and the elongated contact detectors such as 68 which appears at the side of the last section of the arm 6. The static part 4 of the robotic arm is provided with means 60 for fixture to the ground of the pit.

In another embodiment, the robot may be a parallel robot with at least two legs supporting a wrist on a fixed base.

As already mentioned, a coupler is provided at the end of the arm and a complementary part of the coupler is affixed onto the support means for patient. The coupler here is an electro pneumatic one in which a contact is open at an opening command and closed at a closing command so that patient supporting means could be released under control of station 12.

Each one of the patient support means is provided with means (not shown) to firmly maintain the patient in his seating or lying position, especially during all the treatment.

When a given support means for a patient is posted to the docking station, it is recognized by station 12 for controlling the robot on the basis of a numerical identifier. A recognition means to this end could be an electrical switch having a plurality of electrical contacts some of them being connected or not to generate the proper numerical value of the identifier. In another embodiment, recognition means comprises a vision tool having at least a vision camera coupled to a computer running a pattern recognition program to perform recognition of the actual support means which is presented at the docking station and also to control the coupling movement of the robot to the support means.

The control means for the robot of this embodiment are arranged upon three levels:
- a hand-control (not shown on the drawings) at the side of the arm with a terminal which is linked to the actuators of the arm to learn trajectories of the end of the arm under control of an operator;
- a control desk 12 into the treatment room but with protection against X-Ray produced by the imaging devices; and
- Control station 16 with remote control of the robotic arm protected against radiation from tube 14.
   All these three control devices are programmed under a hierarchical organization so that in case of failure, the hand-control at the side of the robotic arm may take control to such an extend that even if the control station 16 and the control desk 12 are disabled, the whole system could still be run.

The robot in this embodiment shows some features aimed to enhance security of the operations

It contains urgent stop-buttons, for stopping any actuator in case of emergency.

Anti-collision bands are mounted at the sides of the robotic arm and at the sides of the patient supporting means.

Additional rigid surfaces are placed to envelop any moving part which is linked to electrical switches.

The program controlling movement of the arm is designed to only permit movement of the patient with given limited speed, acceleration, and angles.

Means are also provided for avoiding unwanted movements whenever treatment is running and/or if power fails. To ensure this, at each joint of the arm, gears are mounted which are always active at the end of movements and/or when power is off. Brakes are provided as well which are also active at zero power. To enhance security within the treatment room, brakes are automatically activated when power is cut off, so that movement of the robot is not possible, unless mechanical coupling means are released.

Instant method takes into account the longitudinal deformation of the robotic arm when it is loaded and/or moving. To this view, movement of the arm is first analyzed with a set of reference loads of known weight. The first moment of movement with its linear and angular acceleration is recorded each time so that a memory of the dynamic response in flexion is memorized. When a patient is placed onto the wrist of the robotic arm, some load detectors and/or accelerometers provided at the wrist are read. Signals produced by load detectors and/or accelerometers are processed to address a bending table or a function which is used to correct and/or adapt movement and localization and/or orientation of the patient at the treatment zone 30. Accordingly, in instant embodiment, the detection signals from the detectors are compared with a dynamic response stored in the memory of the control computer to adapt and correct the dynamic response of the arm.

### General steps of the method

The method according to this embodiment of the invention will now be described in this context.

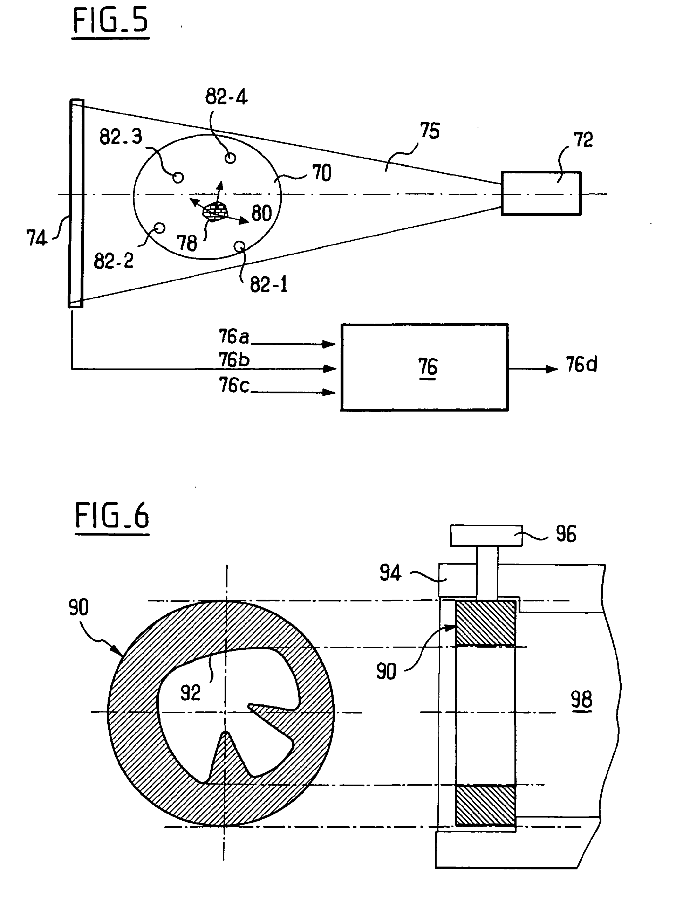

When a patient has been diagnosed with any disease such as a tumor which could be treated with the particle beam (here a proton beam) produced in the treatment room of figure 1, the following occurs:
1. The tumor of the patient to be treated is located and some markers are disposed onto the patient;
2. Imaging of both markers and the body part to be treated is done to produce a database of images with identification of the patient. Imaging is done for example with combined use of X-rays, IRM or PET scan means;
3. Time, duration, axes and power for each designed treatment beam are determined by physicians and ascribed in a database with identification of the patient to help operators in controlling the treatment;
4. At the treatment room, operators teach to the control means the movement of the robotic arm to ensure correct position and orientation of the body part to be treated;
5. At the treatment room, a collimator or diaphragm is formed in a material able to arrest particles of the treatment beam outside the body part to be treated (see Figure 6). Collimator is designed to be mounted at the end of tube 14 connected with particle generator 18, before patient will be treated;
6. If necessary, phantom tank 21 is mounted at the end of the robot arm, is positioned and oriented to intercept the particle beam radiated from tube 14 so that beam dosimetry can be performed;
7. At the time treatment is programmed, the patient is loaded into the treatment room and installed on his support means (chair 8 or couch 7);
8. The support means is moved to the docking station 20 so that reference means 32 or 36 engage with reference means 34 of the docking station 20. For example, a first detector (not shown at the drawings) is provided at the side of the docking station so that the type of support means is detected and recognized to allow control means of the treatment room to set correct parameters corresponding to the type and geometric shape of the support means. Such information permits to precisely specify the area in which the robotic arm should be moved with patient onboard. A second detector (not shown) at the docking station is connected to station 12 to ensure that a signal indicating the beginning of the learned movement of the arm is detected by the control means at station 12.
9. The arm performs the learned movement of its wrist so that it moves the support means from the docking station to the treatment zone 30, the patient having at the end of the movement the computed position (place and orientation) relative to the axis of tube 14;
10. Again, imaging means 40, 42, 44 are used to generate at least two non collinear digital 2D images of the body part to be treated, showing the markers of the body of the patient and the tumor. The images are transmitted to the computer installed at terminal 16. There, the 2D images are processed to calculate actual absolute position of the patient's markers and to compare it with absolute 3D reference positions stored in a database;
11. Accordingly, these newly generated images are compared with the reference images of the database to detect if the markers are present and at the right position and if a predetermined point such as the isocenter of the body part to be treated is located at the correct position (location and orientation) relative to the axis of tube 14. If the positions are correct, treatment beam is fired. If not, robotic arm is controlled with computer 16 to correct the position of the patient. Once the correct position is reached, treatment beam is fired.
12. In many cases, the body part also needs to be irradiated with the beam from another side that is to say with another orientation of the patient with respect to the beam. Thus, the patient is moved in order to have this new orientation, its position is checked and if necessary, modified until the intended new position is reached. Then the beam is fired. The same steps are performed for each firing associated with a new orientation, if any.
13. When all the firings have been done, patient is moved back to the docking station. Its support means is disengaged from fixture means of the robotic arm. The arm goes back to the pit 10 and patient is lead out of the treatment room. The collimator at the end of the tube 14 is eliminated.
When performing the method, the "user" referential (referential well known in robotics which corresponds to the target such as a car door in the case of a painting robot) is put in coincidence with the irradiation beam. The two other robot referentials are the "world" referential (corresponding to the base at the foot of the robot) and the tool referential (the end of the arm holding the tool, here the chair or the bed with the patient). Positioning is performed only once to put the tool (the patient) on the beam and may be automatically readjusted in real time, for example if the target is moving (tracking). Basically, this feedback principle is used to put the tool (the patient!) on the target. With known positioning methods, the patient is maintained into a fixed position (attached on a bed for example), and the beam may be moved around the patient (such as a 360° rotation) without the need to reposition the patient. According to the invention, we can do all we want with the robot without the need to enter the room.

Some of these steps will now be described in greater detail.

### Markers

As announced, a plurality of markers 82 is used.

In one embodiment, markers are inserted in or affixed to the body part of the patient, which is here a head with a skull 70. Each marker could be made in a biocompatible material like gold, titanium or tantalum and be ball-shaped. At the time of placing markers on the skull of the patient, the radiation oncologist or surgeon applies two, three and preferably four markers. The surgeon could insert each marker firmly at the surface of the skull at some particular locations or insert the markers inside the patient head as showed on figure 5, in a known manner.

At Figure 5, four markers 82-1 to 82-4 are inserted in or around a body part 78 to be treated with the proton beam. By way of example, a cancerous tumor of the eye or the head.

Markers may comprise a part which can be imaged and a part adherent to the skin of the patient.

In another embodiment, markers may be affixed onto a part of patient supporting means and preferably onto contention means which are supplied to securely binding patient onto his supporting means. Such markers could be some predetermined parts of the contention means like metallic parts of the links or bands to affix patient, and/or predetermined marks which shape and position are predetermined to generate convenient 2D images when patient is submitted to X-rays imagers as described above.

As explained above, markers in the shape of balls may be settled into the skull or the head of the patient. But there exists a need for another technique which is less invasive.

At figure 9, markers form parts of two plastic tubes which can be inserted into a body natural cavity or orifice such as an ear, the mouth or the nose. Each tube comprises at least one metallic ball which can be imaged with X-ray or other imaging systems like ultrasound waves or infrared imagers. Tubes can also comprise in addition some gadolinium in order to be imaged as well with MRI. In the example of figure 9, two tubes 172 and 174 are settled in position on the head of the patient, in ears. Each tube may contain several X-ray markers 172a and 172b and 174a and 174b. The tumor 170 inside the skull has been schematically drawn and should be treated by applying the proton beam.

At 178 is schematically shown a first X-ray image of the patient's head with his tumor as that image is generated by X-ray imager 72 - 74 at Figure 5. Axis of X-ray beam is shown at 176 and spots of the markers of the two tubes 172 and 174 are referred as squares A' or B'. The space occupied with tumor 78 is represented on the first image 178 at 182 and the spot of the isocenter of the reference coordinate system linked to the tumor has been drawn at 186.

Later, patient is settled on the robotic arm, and then moved and oriented, both the tubes 172 and 174 being in place. At least a second image 180 is then acquired by using one of the X-ray imaging systems 40 - 44 at the treatment room, to correct the patient position before proton beam can be fired at tube 14. The second X-ray image shows that two markers are displaced in A and B and therefore orientation and/or localization should be corrected.

Preferably, instant method comprises setting a prosthesis including at least a marker into at least one natural body cavity. The prosthesis is formed according to the proper shape of the said body cavity (for example by molding directly on the patient's body) and belongs for example to the following group:
- a mouth prosthesis;
- a nose prosthesis; and
- an ear prosthesis.

The marker is a marker for at least one non visible radiation of the following group: X rays and nuclear magnetic resonance and preferably both.

The prosthesis or at least one of the prosthesis may comprise at least two markers spaced from each other and the number of prosthesis set onto the body is at least 2, 3 or 4.

Thus, at least one of the markers may be part of a prosthetic part inserted into the orifice or cavity of the patient's body. By way of example, such a prosthetic part is preferably an audio amplifier inserted into the ear, or an artificial tooth. This prosthesis could also be intended to be fixed against the palate of the patient's mouth. The marker could also be part of a surgical implant on the backspin or on arms or legs.

### Imaging

An important parameter to define the proton beam is the presence of living tissues around the targeted tumor 78. These living tissues should be protected when proton beam is radiated. Therefore, precise orientation of the skull 70 in view of orientation of the proton beam should be defined. A coordinate system associated with the patient and the future beam is used to ensure that parameters of the correct orientation are specified.

During a preparation step, at least two 2D images of the markers and tumor are acquired, which are perpendicular to each other and stored into the database with the patient ID and the treatment session ID. This enables to identify absolute reference positions (place and orientation) of the patient with respect to the room and accordingly the beam, by means of the absolute reference coordinates (x, y and z on three perpendicular axes) of the markers and tumor. On the basis of the recognition of the coordinates of each marker on both 2D images, 3D-absolute reference coordinates of the markers and tumor with respect to the fixed referential of the room are computed and stored in the database. This will permit to configure treatment with both movements of the robotic arm and firing of the proton beam.

More precisely, in order to define the position and orientation of the coordinate system, after the markers have been inserted on the skull 70, at least one 2D image is generated with a X-ray imaging system 72, 74. In instant embodiment, X-ray imaging devices 72, 74 comprise a X-ray source 72 and a detector plate 74 to generate a 2D image of densities of the skull 70. At least both markers 82 should be represented on the numerical image which is stored at the input 76b of the database manager 76. For each acquired 2D image, database manager 76 generates a plurality of parameters comprising:
- Specification of a reference 3D coordinate system comprising a reference point and two reference axes in the 2D image;
- Recognition of the coordinates of each imaged marker in the reference 3D coordinate system;
- Specification of the isocenter of the tumor 78 and at least axis of the specified proton beam;
- storing identifications of the patient and of the proton beam to be applied.

The reference coordinates system 80 comprises three axes and an original point. The original point is denoted "isocenter" and referred to be a preferred point for applying a proton beam when the treatment of the patient is running. Thus, when patient is lying on his support means, he is moved and oriented with the arm, localization and orientation is computed and controlled so that proton beam axis should be aligned and the axis of the proton beam passes through tumor 78 at the well-defined isocenter.

Alternatively the database of 2D images could be acquired on the basis of visible images recorded onto a transparent carrier as is well-known in the art of recording X-ray analogue images. Every parameter specified above is manually or automatically entered in the database manager 76 under responsibility of an operator serving the X-ray imaging system 72, 74. Therefore, in such a manually generated database of 2D images, both "paper-like" files and databases tables are managed with indexes to be able to cross both visual record and numerical records of parameters which have been entered as described above. Peculiarly, isocenter and orientation of the reference coordinate system 80 on the tumor 78 are entered in the database 76 in relation with specification of the proton beam axis that should be applied to the patient later.

If one or two other X-ray imaging systems are provided with the X-ray imaging system 72-74 shown at Figure 5, the output of each of them is connected to the respective input 76a, 76b, 76c at the database manager and the same as described above for x-ray imaging system 72-74 is repeated for the one or two others. However, for a given proton beam, the same isocenter and the same reference coordinate system are used for the two or the three X-ray imaging systems. The plurality of records for the tables loaded in the database manager 76 is controlled on the basis of:
- the name or unique identification ID_Patient of the patient and possibly of his tumors if there is more than one tumor to be treated;
- the identification of the one or more proton beams defined so that the recorded tumors will be treated in subsequent steps and
- the list of the imaged markers 82-1 to 82-4 with their respective coordinates in both 3D reference coordinates system 80 in the tumor 78 to be treated and 2D reference coordinates system on each 2D image entered in the database manager 76.

As explained below, at the beginning of each treatment session, the patient is positioned in the treatment room in front of the tube 14. Markers on the patient are imaged by means of two 2D images and the same computation as above takes place, to calculate this time the actual 3D-absolute coordinates of the markers in the instant actual position (place and orientation) of the patient. Any variation of the patient position is now detected. Precisely, this step makes use of the above-mentioned 3D-absolute reference coordinates of the markers, as previously acquired, and of the actual 3D-absolute actual coordinates of the markers, as newly acquired. If an offset is detected, new positioning of the patient (change of place and/or orientation) is executed with the robotic arm. Once the correct position is obtained, the treatment beam is applied onto the patient according to data.

### Treatment sessions

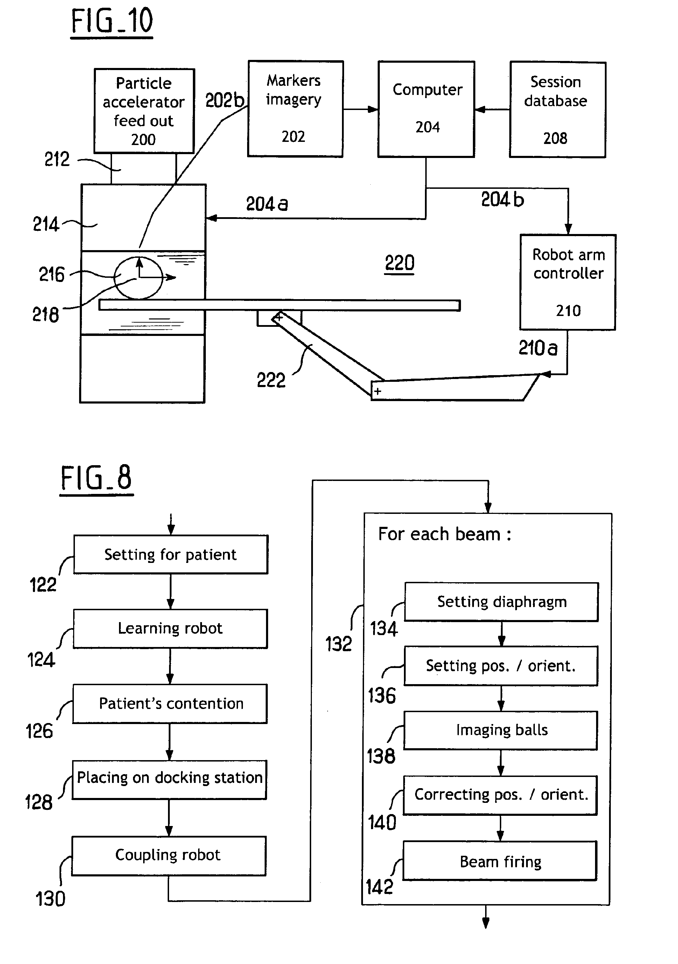

Figure 8 shows the different steps of each treatment session.

At step 122, the patient is prepared to follow his treatment. Then at step 124, operator uses arm control station 16 to enter the movement parameters for the path of the patient supporting means from the docking station to the treatment zone.

After movement and other controls of the robotic arm are loaded in the robot computer, patient is settled on his supporting means. He is attached to it to lessen his freedom of movement with any convenient contention means at step 126.

At step 128, patient supporting means is fixed at the docking station.

At step 130, robotic arm is coupled to the patient supporting means. Technically speaking, such an operation is effected with a stop and lever assembly, one being associated with the wrist of the robotic arm and the other with the patient supporting means. At that time, reference coordinate system at robotic arm is identified to the reference coordinate system associated with the patient supporting means that is to say the room.

At general step 132, each programmed proton beam is fired sequentially as it is explained below.

At step 136, the arm (carrying on its wrist patient supporting means with the patient to be treated) is moved and oriented on the basis of the data recorded in the database 76. It leads the patient from the docking station to a reference point along the central axis of the proton beam which will be fired. At that reference point is settled the isocenter of the tumor to be treated as defined in the database 76.

### Controlling the movements of the arm

Generally speaking, instant method is used for controlling the robot to move the body between initial and final positions by deforming the robot (having six degrees of freedom) along one among a plurality of possible configurations enabling this movement. The deformation configuration is chosen according to at least one cinematic feature of the configuration which could be chosen for example from the group consisting of:
- a level of risks;
- a maximum acceleration;
- a maximum speed; and
- a number of degree of freedom involved by the movement.

Beside, the robot is preferably controlled so that at least a deformable portion of the robot is not deformed during the movement.

In this example, the device is controlled so that the body remains in a predetermined area smaller than any area comprising all the possible locations of the body moved by the robot.

More precisely, in instant embodiment, means are provided for controlling the movements of the arm with the patient supporting means. Such a control means is programmed to command a trajectory of at least the patient supporting means at the end of the robotic arm from its starting position at the docking station to the treatment position in front of the tube 14 so that trajectory is free of colliding risk.

These means cooperates with means for specifying a free movement space at the request of the operator in which any trajectory is calculated. These means for controlling movements of the arm also cooperates with means for classifying a degree of risks of any movement of the arm and to request a choice form the operator (or the computer) in a list of classified movements from the less risky to the most risky movement. In instant case, the means for classifying also comprises means for automatically selecting a movement in the said list of movements with another constraint comprising:
- Maximum speed and/or acceleration (linear and/or circular) authorized for a given patient and/or a given supporting means; and
- Number and, if possible, absolute definition, of constant degrees of freedom of the robotic arm.

For this last constraint, a means is provided for setting some axes of the robot to be non moveable, at least a part of the movement being executed with the other axes varying. It should be understood that, if the robot is replaced with another robotic apparatus like a parallel robot, the same applies for.

### Checking locations of the markers

At step 138, once the patient has reached his position, X-Ray imaging system 40 - 44 acquires one to three 2D images of the skull of the patient at the position (place and orientation) given by the robotic arm before proton beam can be fired.

When the new images have been acquired, they are compared to the data in the database 76 (figure 5) so that actual position of the markers is compared with their reference position as recorded in the database. If the new imaged positions are not in correspondence with the reference positions of the markers, then at step 140, robotic arm modifies localization of the isocenter and orientation of the patient with respect to the central axis of the proton beam to correct the position.

To control this correcting movement, the computer runs a program which reads in the database the data of the reference position of the markers. Then, it reads the data corresponding to the actual position of the markers and deduces control parameters to program movements of the robotic arm to perform a correction of the localization and/or orientation of the isocenter of the tumor.

Generally speaking, instant method comprises the steps of:
- measuring a location of a plurality of positioning items such as the markers; and
- determining from reference locations of the positioning items and the measured locations if at least one of the positioning items moved with respect to at least another one and even with respect to the others

The locations are measured with respect to a given referential such as the room, the body being moveable with respect to this referential.

If one of the positioning items moved, the method comprises the step of determining which it is and positioning the living body by using the positioning items except this one.

To this end, the method comprises, for at least one of the pairs of the positioning items, comparing the distance between their measured positions and the distance between their reference positions, preferably by making the comparison for all the positioning item pairs.

Then the method comprises, for at least a predetermined group of positioning items, calculating a value which takes into account for each item the distance between its measured position and its reference position, and determining if this value is above a predetermined threshold. The number of items in the group is at least 3. But for the group or at least one of the groups, the number of items in the group is less than the total number of positioning items. Calculation and determination are made for a plurality of the groups so that each positioning item belongs to groups comprising different combinations of positioning items.

Some of the values being above their corresponding threshold and some others not, the method comprises determining if at least one of the items belongs to groups associated with the above value without belonging to the other groups, and if yes, determining which it is.

After the body has been moved, the measuring and determining steps are performed again with the previously measured locations used this time as reference locations replacing the former reference locations. Both determination steps may be performed on the same day or on different days.

More precisely, at figure 10, a routine to check if the position of the markers is still correct is illustrated.

At step 150, as explained before, the actual images of the markers are digitized by the computer to obtain sets of 3D coordinates corresponding to the actual position of the markers

At step 154, database manager 76 is addressed with the identification of the patient to be treated.

The 3D coordinates corresponding to the actual position of the markers are compared with the 3D reference coordinates of the markers stored in the database. To this end, for each marker the computer calculates the distance between the actual and reference positions of the marker at step 156.

If this distance equals zero for each marker, each marker is deemed to be in its reference position and not to have moved. The isocenter of the tumor is deduced from the set of the markers actual coordinates. And an "end" operation is performed at Step 162.

At step 158 if an error occurs, that is if one distance does not equal zero, this means that at least one marker has moved and it is signaled to the control computer. Then, the next steps are aimed at identifying the moved marker(s).

In the case of n markers, a loop is generated to monitor each group of any combination of p markers among the n markers with 1 < p < n.

In case of 2 among n markers, the method consists in verifying the distances between all the possible pairs of markers. Thus for each pair, the actual distance is calculated and compared with the distance in the reference position of the markers stored at database manager 76.

In case of 3 or 4 (or more) among n markers, the method consists in trying to match by optimization the reference pattern and the actual pattern. If the match criterium is above a certain threshold, it means that the patterns are different and that one or more markers have moved. Briefly, an example of such a value is as follows. For each marker, the Euclidian distance offset measured between actual and reference positions of the markers is calculated. The value is the sum of the square of these offsets for the markers of the group, or preferably the square root of this sum.

For some of the groups, the value may be null, which means that the markers of this group did not move with respect to each others.

On the contrary, for some other groups, the value may be above zero, which means that at least one the markers of these groups did move with respect to each others. If such an offset is detected, a test is performed to identify the moved marker(s) or even the missing markers if any.

In this view, a plurality of threshold values is used to specify if the offset is acceptable or not. Let {TVᵢ; i} be a set of predetermined threshold values as required according to the number of markers. Let {ODj; j} be a set of all the offset distances measured at the preceding step. A variable counter CPTₖ is incremented each time a logical condition expressed as (ODⱼ > TVᵢ) is verified with marker k.

Two reference values A < B are selected by the operator at station 16. If a given k^{th} counter is greater than a first reference value A (CPTₖ > A), then the presumed k^{th} marker is declared as being misplaced and if CPTₖ > B, then the presumed k^{th} marker is declared as being indefinite.

In a further step, a counting is also performed of all the results onto the p-uplets of these suspect markers. When several counters are greater than the threshold values of A and/or B and only one is below, the marker being actually "vague" or "indefinite" is the one corresponding to the accepted counter.

### Correcting position

Generally speaking, in instant method the robot is controlled to move the body:
- between first and second positions with reference to a fixed point with respect to a base of the apparatus; and
- for performing the correcting movement between the second position and a third position with reference to a point of the second position.

Station 16 on which control program of the treatment session runs cooperates with a means for specifying a degree of resolution at which recognition of the 3D absolute position of each marker is made. When a movement has been computed to nullify offset, it is performed with at least one of the axes of the robotic arm. However, precision of movement is different for each axe of freedom of the arm. In the said database, specifications of various resolutions for each axis of freedom of the robotic arm are stored. Therefore, according to instant embodiment, depending on the resolution of nullification of the offset, the computer identifies which axe(s) of movement should be activated to attain said nullifying resolution

Once the patient is placed in the new position, a new imaging step is performed to check if the new actual position corresponds to the reference position. If necessary, the patient position is once again corrected until the reference position is reached.

The steps for positioning of the patient are repeated for each firing if more than one has been programmed. Indeed different firing may be performed one after the other with different patient orientation. In most cases, the patient will come to the treatment room at different days (for example one session in each day, each session including several firings).

According to instant method, data and/or images of the markers and patient are all stored into the database in relation with the specifying treatment data of the patient. Therefore, when treatment is resumed, new positioning may be performed on the basis of the stored data of any combination of older sessions. Preferably, it is controlled on the basis of the stored data of the last session because it is more advantageous to compare the new 3D data of the markers with the 3D positions stored the last time the treatment has been conducted. Thus, these positions are used as the new reference positions. Accordingly, time and operations are spared if some markers have moved at least two sessions before.

### Firing the beam

Once the correct position is reached, the beam is fired with the patient not moving with respect to the beam.

Instant invention may be used to irradiate a large target with at least one beam having a smaller cross-section than that of the target. Therefore, under control of station 16, a convenient sequence of movements and orientations of the patient is performed in relation with the set of proton beams which have been programmed.

Means are provided for controlling relative movement of the patient and/or the at least one beam according to a predetermined trajectory to cover the entire space occupied by the at least one tumor. Means could also be provided for varying localization and/or orientation of the patient relative to the at least one treatment beam with external parameters like beam dose rate and duration.

Beside, the invention may be performed such that during firing the beam, place and/or orientation of the patient with respect to the beam change continuously to treat the targeted tumor.

The invented method allows to gain time to position the patient before the or each firing of a treatment session on a given day and between two sessions.

An important feature is that absolute 3D reference positions of the markers which has been setup at the preparing session may be renewed using the last 3D positions of the markers to date. Further, it is possible to store in the database the control parameters of the movement to move patient from the docking station to the treatment zone as it is programmed at one of the latter sessions of treatment, and then to compute offset movement to correct the final position and orientation of the patient on the basis of the instant 3D positions of the markers acquired at the time of the said 2D images are acquired before firing treatment beam.

The method of the invention may be performed with an isocentric rotation of the patient under control of the computer 16. In such a treatment, once patient has been settled onto the support means, the patient isocenter which is linked to the robot is kept without correcting its localization once again. Therefore, the same isocenter being defined at computer 16, a plurality of beams can be fired in sequence, only one rotation movement of the patient around isocenter being performed between two successive firings.

At Figure 10, an alternate embodiment of the apparatus of the invention has been illustrated in which the source of treatment is moveable around the living body under treatment.

At an output port of the beam accelerator (not shown), a particle accelerator feed out device 200 is provided which is connected to convenient means 212 to feed at least one moving particle beam which is mounted to be rotatable inside a gantry 214.

Furthermore, at each point for feeding a proton beam, a system of magnetic lenses is provided to direct central axis of the proton beam with a given angle, the value of which can be determined by electronic control means in relation with any relative movement programmed at the computer unit 204 to localize and/or orientate isocenter and coordinate system of the patient, with reference to the isocenter and a first reference coordinates system associated with gantry 214.

At the center of gantry 214, a hole is provided through which patient under treatment will be placed at the location shown at sphere 216. Patient is loaded from a docking station as described above toward the treatment place 216. To move him from the docking station (not shown) to the treatment place 216, he is loaded onto a couch 220 which has been connected to a convenient pneumatic coupler at the end of the wrist of a robotic arm 222.

Robot arm controller 210 comprises a trajectory memory which is addressed with particular parameters which are loaded in it during an initialization session before patient is loaded onto the couch 220. With its memory, controller 210 has been learned to control couch 220 and robotic arm 222 to follow a best fit trajectory from the docking station to the treatment place 216 as described above. To move arm 220, controller 210 supplies control signals to actuators of arm 222 via a control link 210a.

Of course, instant invention may be modified in many ways without departing from its scope.

## Claims

1. A method for positioning a body (70), such as a living body, **characterized in that** it comprises:
- measuring a location of a plurality of positioning items (82, 172a, 172b, 174a, 174b); and
- determining from reference locations of the positioning items and the measured locations if at least one of the positioning items moved with respect to at least another one.

2. A method according to the preceding claim, **characterized in that** the locations are measured with respect to a given referential, the body being moveable with respect to this referential.

3. A method according to any of the preceding claims, **characterized by** determining if at least one of the positioning items moved with respect to the others.

4. A method according to any of the preceding claims, **characterized by**, if one of the positioning items moved, determining which it is and positioning the living body by using the positioning items except this one.

5. A method according to any of the preceding claims, **characterized by**, for at least one of the pairs of the positioning items, comparing the distance between their measured positions and the distance between their reference positions.

6. A method according to the preceding claim, **characterized by** making the comparison for all the pairs of positioning items.

7. A method according to any of the preceding claims, **characterized by**, for at least a predetermined group of positioning items, calculating a value which takes into account for each item the distance between its measured position and its reference position, and determining if this value is above a predetermined threshold.

8. A method according to the preceding claim, **characterized in that** for the group or at least one of the groups, the number of items in the group is less than the total number of positioning items.

9. A method according to any of claims 7 to 8, **characterized in that** calculation and determination are made for a plurality of the groups so that each positioning item belongs to groups comprising different combinations of positioning items.

10. A method according to any of claims 7 to 9, **characterized in that**, some of the values being above their corresponding threshold and some others not, determining if at least one of the items belongs to groups associated with the above value without belonging to the other groups, and if yes, determining which it is.

11. A method according to any of the preceding claims, **characterized in that**, after the body has been moved, the measuring and determining steps are performed again with the previously measured locations used as reference locations replacing the former reference locations.

12. A method according to the preceding claim, **characterized in that** both determination steps are performed on the same day.

13. A method according to claim 11, **characterized in that** both determination steps are performed on different days.

14. A device (12, 16) for positioning a body (70), such as a living body, comprising means (40, 42, 72, 74) for measuring a location of a plurality of positioning items (82, 172a, 172b, 174a, 174b), **characterized in that** it comprises means (16) for determining from reference locations of the positioning items and the measured locations if at least one of the positioning items moved with respect to at least another one.

15. An apparatus (10, 12, 16) for moving a body, **characterized in that** it comprises a device according to the preceding claim.

16. A method for positioning a living body **characterized in that** it comprises setting a prosthesis (172, 174) including at least a marker (172a, 172b, 174a, 174b) to both X-rays and nuclear magnetic resonance, into at least one natural body cavity.

17. A method according to the preceding claim, **characterized in that** the prosthesis is formed according to the proper shape of the said body cavity.

18. A method according to any of claims 16 or 17, **characterized in that** the prosthesis or at least one of the prosthesis belongs to the following group:
- a mouth prosthesis;
- a nose prosthesis; and
- an ear prosthesis.

19. A method according to any of claims 16 to 18, **characterized in that** the prosthesis or at least one of the prosthesis comprises at least two markers (172a, 172b, 174a, 174b) spaced from each other.

20. A method according to any of claims 16 to 19, **characterized in that** the number of prosthesis set onto the body is at least 2, 3 or 4.

21. A prosthesis (172, 174) shaped to be set into a natural cavity of a living body, **characterized in that** it includes at least a marker to both X-rays and nuclear magnetic resonance.

22. A method for controlling a device (10) arranged to move a body, such as a living body (70), between initial and final positions by deforming the device along one among a plurality of possible configurations enabling this movement, **characterized in that** the deformation configuration is chosen according to at least one cinematic feature of the configuration.

23. A method according to the preceding claim, **characterized in that** the feature or at least one of the features is chosen from the group consisting of:
- a level of risks;
- a maximum acceleration;
- a maximum speed; and
- a number of degree of freedom involved by the movement.

24. A method according to any of claims 22 to 23, **characterized in that** the device is controlled so that the body remains in a predetermined area (30) smaller than any area comprising all the possible locations of the body moved by the device (10).

25. An apparatus (10) arranged for moving a body (70), such as a living body, between initial and final positions by deforming itself along one among a plurality of possible configurations enabling this movement, the apparatus comprising control means, **characterized in that** the control means (12, 16) are arranged to enable to choose the deformation configuration according to at least one cinematic feature of the configuration.

26. A method according to any of claims 1 to 13, 16 to 20 or 22 to 24, **characterized in that** at least one two-dimensional image of the body (70) is taken.

27. A method according to any of claims 1 to 13, 16 to 20, 22 to 24 or 26, **characterized by** determining three dimensional coordinates of some points of the body (70).

28. A treatment method, such as a method for treating a tumor by radiotherapy, **characterized in that** it comprises a method according to any of claims 1 to 13, 16 to 20, 22 to 24, 26 or 27.

29. An apparatus (10, 12, 16) for moving a body (70) with respect to a base of the apparatus, the apparatus comprising holding means (6) for holding the body during a movement, **characterized in that** it comprises at least two different items (21, 7, 8), at least one (7,8) of which being arranged to carry the body, the apparatus being arranged to detachably fix any of the items to the holding means.

30. An apparatus according to the preceding claim, **characterized in that** two of the items (7,8) or both items are arranged to carry the body (70).

31. An apparatus according to claim 29 or 30, **characterized in that** at least one (21) of the items is a test device not arranged to carry the body.

32. An apparatus according to any of claims 15, 25, and 29 to 31 **characterized in that** it comprises means for treating a tumor (78) by radiotherapy.

33. An apparatus according to any of claims 15, 25 and 29 to 32 **characterized in that** it comprises a gantry (214).
